Europäisches Patentamt

European Patent Office

Office européen des brevets

(11) Numéro de publication : **0 471 632 B1**

# (12) FASCICULE DE BREVET EUROPEEN

(45) Date de publication du fascicule du brevet :
**17.05.95 Bulletin 95/20**

(51) Int. Cl.⁶ : **G04D 7/00,** G01N 19/04,
A44C 17/04

(21) Numéro de dépôt : **91440064.3**

(22) Date de dépôt : **01.08.91**

(54) **Procédé et appareil pour contrôler des pièces d'horlogerie et/ou de bijouterie.**

(30) Priorité : **17.08.90 FR 9010439**

(43) Date de publication de la demande :
**19.02.92 Bulletin 92/08**

(45) Mention de la délivrance du brevet :
**17.05.95 Bulletin 95/20**

(84) Etats contractants désignés :
**AT BE CH DE DK ES FR GB GR IT LI LU NL SE**

(56) Documents cités :
**CH-A- 378 243**
**DE-A- 3 441 805**
**FR-A- 1 276 325**
**GB-A- 1 523 295**

(73) Titulaire : **SOCIETE DE FABRICATION MATY(
SFM) SARL**
**2, rue Gay Lussac**
**F-25009 Besançon Cédex (FR)**

(72) Inventeur : **Tyrode, Alain**
**2, rue Gay Lussac**
**F-25009 Besancon Cedex (FR)**

(74) Mandataire : **Nithardt, Roland**
**CABINET NITHARDT & BURKARD**
**12 rue du 17 Novembre**
**Boite Postale 1445**
**F-68071 Mulhouse Cédex (FR)**

## Description

La présente invention concerne un procédé de contrôle de la fixation d'éléments rapportés sur des pièces d'horlogerie et/ou de bijouterie, notamment de pierres précieuses ou semi-précieuses montées par sertissage, dans lequel l'on soumet individuellement ces pièces à au moins un train de vibrations, l'on capte les vibrations résultantes au moyen d'un capteur, l'on analyse les signaux générés par ce capteur, ces signaux étant, le cas échéant, perturbés par des chocs dus à un ou plusieurs éléments mal fixés, et l'on détecte une pièce défectueuse en constatant l'existence de signaux perturbés.

Elle concerne également un appareil pour la mise en oeuvre du procédé ci-dessus.

L'un des problèmes qui se pose lors de la fabrication artisanale ou industrielle de pièces d'horlogerie et/ou de bijouterie comportant des éléments rapportés et notamment des pierres précieuses ou semi-précieuses montées par sertissage, est celui du contrôle efficace de la fixation ou de la tenue desdits éléments rapportés.

Le brevet CH-378 243 décrit un procédé de mise à l'épreuve de pièces d'horlogerie face à diverses perturbations extérieures. Les contrôles réalisés sur un établi circulaire rotatif comportant plusieurs postes de contrôle du comportement d'une pièce d'horlogerie soumise à certaines contraintes telles que variation de pression ambiante, chocs, vibrations, etc sont non instantannés et les mesures effectives sont relevées individuellement par l'opérateur ayant une position fixe par rapport à cet établi. Le brevet DE-3 441 805 décrit un dispositif de mise à l'épreuve d'ensembles amortisseurs permettant de vérifier les liaisons métal/caoutchouc, en appliquant des vibrations à une fréquence fixe ou variable et en comparant les vibrations résultantes aux vibrations initiales pour détecter un éventuel défaut de liaison si les vibrations résultantes sont perturbées.

Il n'existe donc actuellement aucun appareil simple permettant de faire une vérification efficace de la fixation des éléments rapportés sur des pièces d'horlogerie et/ou de bijouterie et plus particulièrement de contrôler si une ou plusieurs pierres précieuses ou semi-précieuses sont bien ou mal serties.

La présente invention se propose de pallier cet inconvénient en mettant à disposition de l'industrie un procédé de contrôle efficace et rapide ainsi qu'un appareil d'utilisation simple et de construction économique pour la mise en oeuvre de ce procédé.

Dans ce but, le procédé selon l'invention est caractérisé en ce que l'on soumet les pièces à au moins un train de vibrations de fréquence déterminée en fonction du type de pièce à contrôler, en ce que ce train de vibrations a une fréquence variable entre deux valeurs limites déterminées, en ce que les vibrations sont du type sinusoïdal à basse fréquence, en ce que l'on compare les signaux à des valeurs de consigne et en ce que l'on commande un organe indicateur pour signaler la présence d'une pièce défectueuse.

L'organe indicateur comporte avantageusement au moins un voyant lumineux.

Selon une variante de réalisation, l'on visualise les signaux sur un écran cathodique.

Pour contrôler les pièces, on les fixe de préférence rigidement au moyen d'une pince vibrante.

L'on oriente de préférence la pince par rapport à l'axe des vibrations de manière à obtenir une sensibilité maximale des signaux du capteur.

Selon une variante avantageuse, l'on modifie l'orientation de la pince en cours de contrôle.

Dans ce but également, l'appareil selon l'invention est caractérisé en ce que les moyens pour soumettre les pièces individuellement à des vibrations sont agencés pour générer au moins un train de vibrations de fréquence déterminée en fonction du type de pièce à contrôler, en ce que le capteur est un accéléromètre, en ce que lesdits moyens comportent un vibreur agencé pour engendrer des vibrations sinusoïdales à basse fréquence, en ce que les moyens pour détecter une pièce défectueuse comportent au moins un comparateur agencé pour comparer les signaux perturbés à des valeurs de consigne et en ce qu'il comporte un organe indicateur pour signaler la présence d'une pièce défectueuse.

Cet organe indicateur comporte avantageusement au moins un dispositif visuel, tel qu'un voyant lumineux. Selon un autre mode de réalisation, l'organe indicateur peut comporter un dispositif acoustique.

Selon une variante de réalisation, l'appareil comporte un écran de visualisation des signaux.

Pour tenir la pièce, l'appareil comporte avantageusement une pince pour fixer les pièces à contrôler.

La pince est de préférence orientable par rapport à l'axe des vibrations générées par le vibreur.

De préférence, le vibreur comporte un bloc cylindrique rigide et un support vibrant relié à ce bloc par une rotule et à la pince par un axe.

La présente invention sera mieux comprise en référence à la description d'un exemple de réalisation et du dessin annexé dans lequel :

- la figure 1 représente une vue d'ensemble de l'appareil selon l'invention,
- la figure 2 représente une vue agrandie d'une partie de l'appareil selon l'invention,
- la figure 3 représente une vue schématique de la partie du circuit électronique permettant de générer les vibrations, et
- la figure 4 représente une vue schématique de la partie du circuit électronique permettant d'effectuer les mesures.

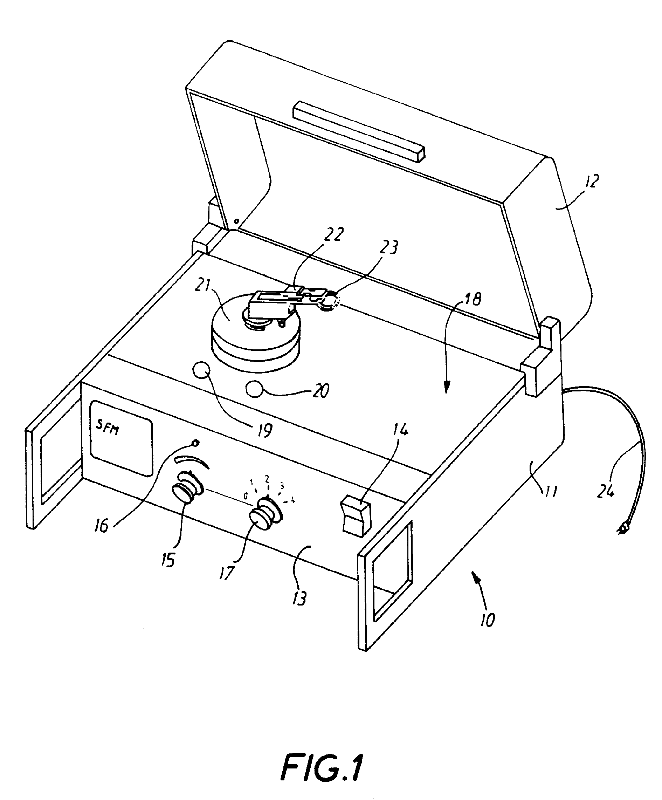

En référence à la figure 1, l'appareil représenté se compose d'un coffret 10 composé d'une base 11 et d'un capot de protection 12, à l'intérieur duquel sont montés les composants essentiels permettant d'assurer les fonctions de cet appareil. Sur la face frontale 13 de la base 11 sont montés un interrupteur de commande 14 de marche et d'arrêt, un sélecteur de calibration 15 associé à un voyant lumineux 16 et un sélecteur de sensibilité 17.

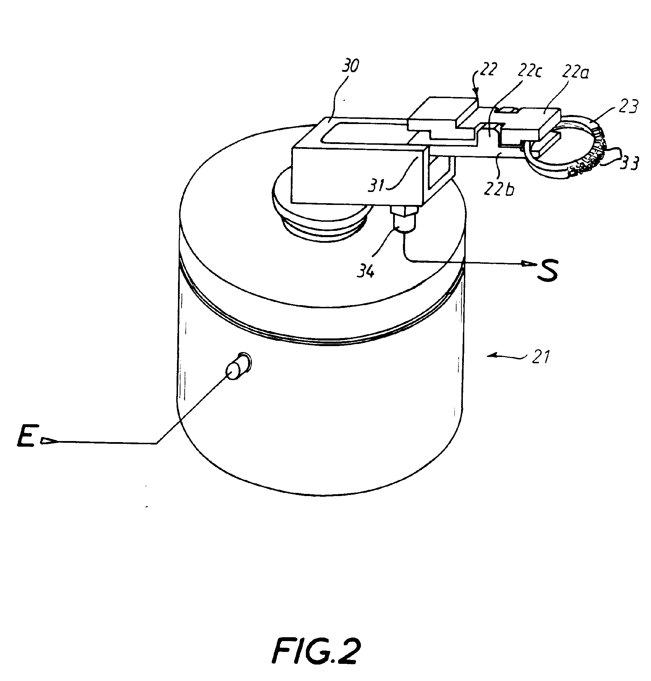

Sur la face supérieure 18 de la base 11 de l'appareil, apparaissent deux voyants lumineux 19 et 20 qui constituent des organes indicateurs destinés à montrer à l'utilisateur si une pièce est ou non défecteuse. Ces organes indicateurs peuvent également être remplacés par un écran cathodique de visualisation, intégré au capot de protection 12 sur sa face intérieure ou extérieure, ou utilisé comme unité périphérique extérieure à l'appareil. Ladite face supérieure 18 comporte également l'ensemble composé du vibreur 21 et de la pince 22 de maintien des pièces à tester 23 qui sont représentés plus en détail par la figure 2. Un cordon d'alimentation 24 permet de raccorder l'appareil au secteur.

Cette figure montre le vibreur 21 qui se présente sous la forme d'un bloc cylindrique qui comporte à son extrémité supérieure un support vibrant 30 sur lequel est montée la pince 22. Cette pince est reliée au support vibrant 30 au moyen d'un axe horizontal 31 qui lui permet d'être positionnée selon des orientations variables. La pince proprement dite comporte deux bras 22a et 22b articulés autour d'un axe 22c, et qui sont agencés pour tenir fermement une pièce à tester, telle qu'une bague 23 sur laquelle sont montées des pierres ou d'autres éléments sertis 33. Un accéléromètre 34 qui constitue un capteur de vibrations, est fixé sous le support vibrant 30.

L'ensemble vibreur 21 et accéléromètre 34 sont des composants du commerce, par exemple vendus par la société BRÜEL et KJAER. Le vibreur utilisé porte la référence 4810 et son équipage mobile à une masse faible, de l'ordre de 18 g. L'accéléromètre dont la référence est 4375 a également une masse faible, de l'ordre de 2,4 g et présente une bande passante de 16,5 KHz.

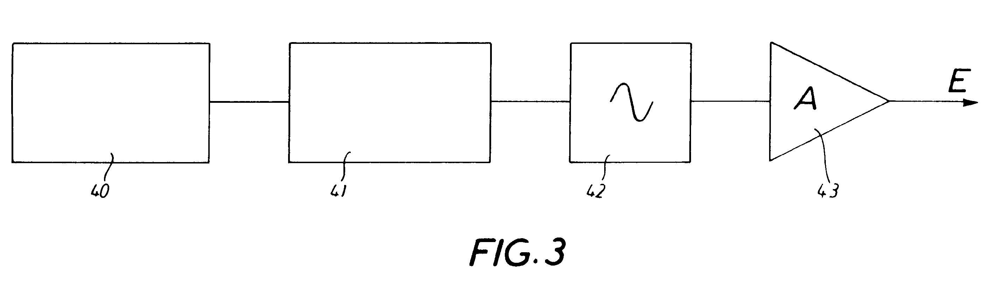

La figure 3 illustre le circuit électronique d'excitation du vibreur. Il comporte un oscillateur 40 de très basse fréquence (environ 0,5 Hz) qui génère un signal pilote à la fréquence de 0,5 HZ. Cet oscillateur est couplé à un oscillateur 41 qui génère un signal pseudo-sinusoïdal de fréquence variable entre 150 et 250 Hz dont la variation est pilotée par l'oscillateur 40. Un filtre 42 élabore un signal sinusoïdal pur à partir du signal de sortie de l'oscillateur 41. Un amplificateur 43 amplifie le signal de sortie afin d'obtenir la puissance nécessaire à la commande du vibreur 21.

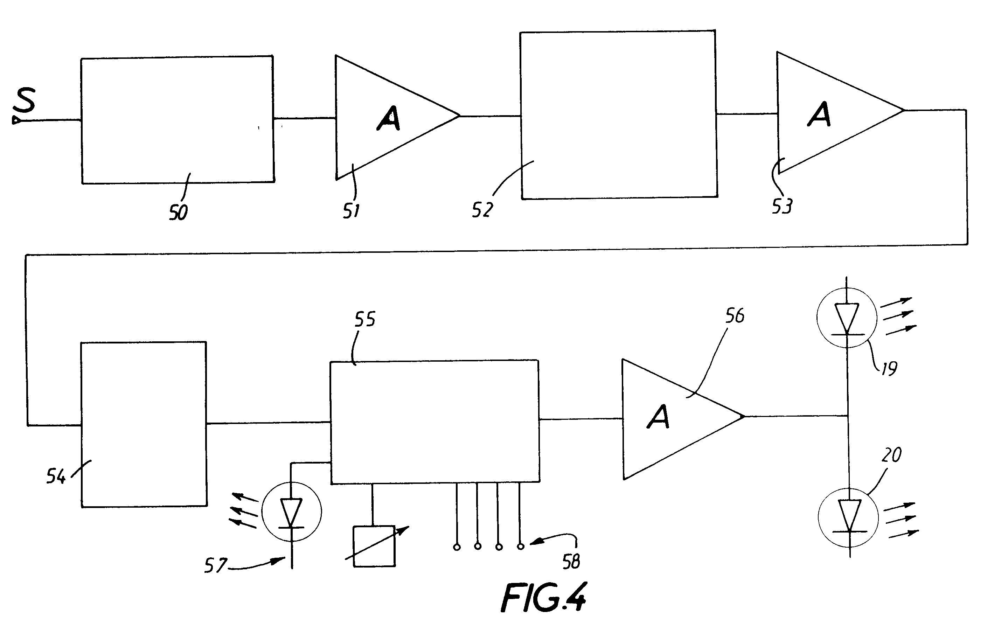

La figure 4 illustre le circuit électronique de mesure qui comporte un bloc 50 adaptateur-préaccentuateur, qui adapte l'impédance d'entrée à celle du capteur, en l'occurence l'accéléromètre et amplifie les fréquences élevées, un amplificateur 51 qui amplifie le signal de sortie de l'adaptateur-préaccentuateur 50, un filtre actif 52 passe haut qui supprime les basses fréquences du signal et en particulier la fréquence d'excitation du vibreur, un second amplificateur 53 qui amplifie de façon ajustable les fréquences alternatives et élimine la composante continue du signal, un intégrateur actif 54 qui intègre le signal de sortie de l'amplificateur 53 afin de générer un signal continu

$$V = -0,5 \int_{t_1}^{t_2} Ve\, dt,$$

un comparateur actif 55 qui compare la sortie de l'intégrateur actif 54 à un niveau de consigne sélectionné et un troisième amplificateur 56 dont la sortie est connectée aux deux voyants lumineux 19 et 20 et qui amplifie le signal de sortie du comparateur actif 55 pour commander la visualisation du résultat du test. L'un des voyants est par exemple vert et fournit un signal indiquant que la pièce n'est pas défectueuse. L'autre peut être rouge et signale un défaut de sertissage. Les composants 57, sont couplés à la commande de calibration constituée par le sélecteur. Les entrées 58 du comparateur actif 55 sont connectées au sélecteur de sensibilité 17.

Pour effectuer des contrôles au moyen de l'appareil décrit ci-dessus, on le met sous tension, puis on le calibre et on choisit le niveau de sélection, c'est-à-dire la sévérité des contrôles. Pour ces opérations, la pièce à contrôler n'est pas montée dans la pince.

Après ces opérations préalables, on met les pièces à contrôler une à une dans la pince. Cette pince est orientable par rapport à l'axe des vibrations qui est l'axe vertical.

La mise en place et le retrait des pièces peuvent être effectués manuellement ou automatiquement. Dans ce dernier cas, la séparation des pièces non acceptées de celles qui sont conformes, peut se faire de manière automatique.

Pour chaque type de pièce à contrôler, on sélectionne une gamme de fréquences et on effectue un balayage de ces fréquences pour engendrer un train de vibrations auquel on soumet lesdites pièces. Les valeurs limites sont déterminées empiriquement et tiennent compte notamment de la masse des pièces ou de leur forme.

Les pièces défectueuses sont soit détectées au moyen des voyants lumineux 19 et 20, soit par visualisation sur l'écran cathodique. Lorsque l'on utilise un écran, les signaux peuvent être analysés d'une manière plus détaillée.

L'invention n'est pas limitée aux applications mentionnées ci-dessus, mais peut s'étendre à toutes les utilisations où la fixation de composants doit être testée et la détection pourrait également se faire par une méthode optique, par analyse d'image, ou tout autre moyen.

## Revendications

1.  Procédé de contrôle de la fixation d'éléments rapportés sur des pièces d'horlogerie et/ou de bijouterie, notamment de pierres précieuses ou semi-précieuses montées par sertissage, dans lequel l'on soumet individuellement ces pièces à des vibrations, l'on capte les vibrations résultantes au moyen d'un capteur, l'on analyse les signaux générés par ce capteur, ces signaux étant, le cas échéant, perturbés par des chocs dus à un ou plusieurs éléments mal fixés, et l'on détecte une pièce défectueuse en constatant l'existence de signaux perturbés, caractérisé en ce que l'on soumet les pièces à au moins un train de vibrations de fréquence déterminée en fonction du type de pièce à contrôler, en ce que ce train de vibrations a une fréquence variable comprise entre deux valeurs limites déterminées, en ce que les vibrations sont du type sinusoïdal à basse fréquence, en ce que l'on compare les signaux à des valeurs de consigne et en ce que l'on commande un organe indicateur pour signaler la présence d'une pièce défectueuse.

2.  Procédé selon la revendication 1, caractérisé en ce que l'organe indicateur comporte au moins un voyant lumineux.

3.  Procédé selon la revendication 1, caractérisé en ce que l'on visualise les signaux sur un écran cathodique.

4.  Procédé selon la revendication 1, caractérisé en ce que les pièces à contrôler sont fixées rigidement au moyen d'une pince.

5.  Procédé selon la revendication 4, caractérisé en ce que l'on oriente la pince par rapport à l'axe des vibrations de manière à obtenir une sensibilité maximale des signaux du capteur.

6.  Procédé selon la revendication 4, caractérisé en ce que l'on modifie l'orientation de la pince en cours de contrôle.

7.  Appareil pour contrôler la fixation d'éléments rapportés sur des pièces d'horlogerie et/ou de bijouterie, notamment de pierres précieuses ou semi-précieuses montées par sertissage, comportant des moyens pour soumettre individuellement ces pièces à des vibrations, un accéléromètre servant de capteur pour capter les vibrations résultantes, des moyens pour analyser les signaux générés par ce capteur, ces signaux étant, le cas échéant, perturbés par des chocs dus à un ou plusieurs éléments mal fixés, et des moyens pour détecter une pièce défectueuse agencés pour constater l'existence de signaux perturbés, caractérisé en ce que les moyens pour soumettre les pièces individuellement à des vibrations sont agencés pour générer au moins un train de vibrations de fréquence déterminée en fonction du type de pièce à contrôler, en ce que ces moyens comportent un vibreur (21) agencé pour engendrer des vibrations sinusoïdales à basse fréquence, en ce que les moyens pour détecter une pièce défectueuse comportent au moins un comparateur agencé pour comparer les signaux perturbés à des valeurs de consigne et en ce que l'appareil comporte un organe indicateur pour signaler la présence d'une pièce défectueuse.

8.  Appareil selon la revendication 7, caractérisé en ce que l'organe indicateur comporte au moins un dispositif visuel.

9.  Appareil selon la revendication 8, caractérisé en ce que le dispositif visuel comprend au moins un voyant lumineux (19, 20).

10. Appareil selon la revendication 7, caractérisé en ce que l'organe indicateur comporte au moins un dispositif acoustique.

11. Appareil selon la revendication 7, caractérisé en ce qu'il comporte un écran de visualisation des signaux.

12. Appareil selon la revendication 7 caractérisé en ce qu'il comporte une pince (22) pour fixer les pièces à contrôler (23).

13. Appareil selon la revendication 12, caractérisé en ce que la pince (22) est orientable par rapport à l'axe des vibrations générées par le vibreur (21).

14. Appareil selon la revendication 7, caractérisé en ce que le vibreur (21) comporte un bloc cylindrique rigide et un support vibrant (30) relié à ce bloc par une rotule et à la pince (22) par un axe (31).

## Patentansprüche

1.  Verfahren zur Kontrolle der Befestigung von Elementen im Uhren- und/oder Juwelierhandwerk, insbesondere für Edel- oder Halbedelsteine, die durch Einfassen befestigt sind, indem man diese Teile individuell Vibrationen aussetzt, die entstehenden Vibrationen durch einen Sensor auffängt und die durch den Sensor erzeugten Signale analysiert, wobei diese Signale, gegebenenfalls, durch Stösse gestört werden, die von einem oder mehreren ungenügend befestigten Elementen verursacht wurden und man ein fehlerhaftes Teil erfassen kann, indem man das Vorhandensein von gestörten Signalen feststellt, gekennzeichnet dadurch, dass man diese Teile mindestens einer Serie von Vibrationen aussetzt, deren Frequenz von der Beschaffenheit des zu untersuchenden Teils bestimmt wird, und dadurch dass diese Serie von Vibrationen eine variable Frequenz zwischen zwei bestimmten Grenzwerten hat, dass die Vibrationen sinusartig mit niedriger Frequenz sind, dass man die Signale mit den Einstellwerten vergleicht und dass man eine Anzeigevorrichtung betätigt, um die Anwesenheit eines fehlerhaften Teils anzuzeigen.

2. Verfahren gemäss Anspruch 1, gekennzeichnet dadurch, dass die Anzeigevorrichtung mindestens eine Leuchtanzeige enthält.

3. Verfahren gemäss Anspruch 1, gekennzeichnet dadurch, dass man die Signale auf einem Kathodenstrahlbildschirm darstellt.

4. Verfahren gemäss Anspruch 1, gekennzeichnet dadurch, dass die zu untersuchenden Teile fest mittels einer Klemmvorrichtung befestigt sind.

5. Verfahren gemäss Anspruch 4, gekennzeichnet dadurch, dass man die Klemmvorrichtung in Bezug auf die Vibrationsachse orientiert, um so eine maximale Sensibilität der Sensorsignale zu erhalten.

6. Verfahren gemäss Anspruch 4, gekennzeichnet dadurch, dass man die Orientierung der Klemme im Laufe der Untersuchung verändert.

7. Gerät zur Untersuchung der Befestigung von Elementen in Teilen aus dem Uhren- und/oder Juwelierhandwerk, insbesondere Edel- oder Halbedelsteine die durch Einfassen befestigt sind, das Vorrichtungen enthält, um diese Teile individuell Vibrationen auszusetzen, wobei ein Beschleunigungsmesser als Sensor dient, um die entstehenden Vibrationen aufzufangen, Vorrichtungen um die von diesem Sensor erzeugten Signale zu analysieren, wobei diese Signale, gegebenenfalls, durch Stösse gestört werden, die von einem oder mehreren ungenügend befestigten Elementen verursacht wurden und Vorrichtungen zur Erfassung von fehlerhaften Teilen durch Feststellen der Anwesenheit von gestörten Signalen, gekennzeichnet dadurch, dass die Vorrichtungen um die individuellen Teile Vibrationen auszusetzen so eingerichtet sind, dass sie mindestens eine Serie von Vibrationen erzeugen, deren Frequenz durch die Beschaffenheit des zu untersuchenden Teils bestimmt wird, dass diese Vorrichtungen einen Vibrator (21) enthalten, der sinusartige Vibrationen niedriger Frequenz erzeugt, dass die Vorrichtungen zur Erfassung fehlerhafter Teile mindestens einen Komparator enthalten, der die gestörten Signale mit den Einstellwerten vergleicht und dass das Gerät eine Anzeigevorrichtung enthält, um das Vorhandensein eines fehlerhaften Teils anzuzeigen.

8. Gerät gemäss Anspruch 7, gekennzeichnet dadurch, dass die Anzeigevorrichtung mindestens eine Sichtvorrichtung enthält.

9. Gerät gemäss Anspruch 8, gekennzeichnet dadurch, dass die Sichtvorrichtung mindestens eine Leuchtanzeige (19, 20) enthält.

10. Gerät gemäss Anspruch 7, gekennzeichnet dadurch, dass die Anzeigevorrichtung mindestens eine Akustikvorrichtung enthält.

11. Gerät gemäss Anspruch 7, gekennzeichnet dadurch, dass es einen Bildschirm zur Darstellung der Signale enthält.

12. Gerät gemäss Anspruch 7, gekennzeichnet dadurch, dass es eine Klemme (22) enthält, mit der die zu untersuchenden Teile (23) befestigt werden.

13. Gerät gemäss Anspruch 12, gekennzeichnet dadurch, dass die Klemme (22) verstellbar ist, in Bezug auf die Achse der vom Vibrator (21) erzeugten Vibrationen.

14. Gerät gemäss Anspruch 7, gekennzeichnet dadurch, dass der Vibrator (21) einen starren zylindrischen Block enthält und einen vibrierenden Träger (30), der mit diesem Block durch ein Kugelgelenk und mit der Klemme (22) durch eine Achse (31) verbunden ist.

## Claims

1. A process for testing of fastening of elements mounted in clockwork mechanisms and/or jewellery, in particular precious or semi-precious stones fastened by crimping, by subjecting each element individually to a given vibration, the return vibrations being detected by a sensor, the signal generated by which is then analysed, these signals being, in given cases, irregular due to the shock effect caused by one or several badly mounted elements, the defective element being localised by analysis of this disturbed signal, characterised in that the elements are subjected to at least one vibration source at a frequency determined by the type of element to be tested, in that the vibration source has a frequency variable with two set limit values, in that the vibration is sinusoidal at low frequency, in that one compares the signals with the set values and in that one activates a indication facility to signal the presence of a defective element.

2. Process according to claim 1, characterised in that the indication facility comprises at least one light indicator.

3. Process according to claim 1, characterised in that the signals are displayed on a cathode ray screen.

4. Process according to claim 1, characterised in that the element to be tested is firmly fastened by means of a clamp.

5. Process according to claim 4, characterised in that the clamp is aligned with axis of vibration in order to obtain maximum sensitivity of sensor signals.

6. Process according to claim 4, characterised in that the clamp alignment is modified during test.

7. Apparatus for testing of fastening of elements mounted in clockwork mechanisms and/or jewellery, in particular precious or semi-precious stones fastened by crimping, equipped with a means of subjecting each element individually to a given vibration, an accelerometer used to detect the return vibrations, a means of analysing the signals generated by this sensor, these signals being, in given cases, irregular due to the shock effect caused by one or several badly mounted elements, and a means of detecting a defective element designed to detect the existence of a disturbed signal, characterised in that the means for subjecting the elements individually to a given vibration is designed to generate at least one vibration source at a frequency determined by the type of element to be tested, in that this means comprises a vibrator (21) designed to produce a low frequency sinusoidal vibration, and that this means for detecting a defective element comprises at least one comparator designed to compare the disturbed signals with the set values and in that the apparatus comprises an indication facility to signal the presence of a defective element.

8. Apparatus according to claim 7, characterised in that the indication facility comprises at least one visual display facility.

9. Apparatus according to claim 8, characterised in that the visual display facility comprises at least one light indicator (19, 20).

10. Apparatus according to claim 7, characterised in that the indication facility comprises at least one acoustic device.

11. Apparatus according to claim 7, characterised in that it comprises a screen for display of signals.

12. Apparatus according to claim 7, characterised in that it comprises a clamp (22) for fastening element to be tested (23).

13. Apparatus according to claim 12, characterised in that the clamp (22) may be swivelled in respect to axis of vibration generated by vibrator (21).

14. Apparatus according to claim 7, characterised in that the vibrator (21) is composed of a rigid cylindrical block and a vibrating base (30) connected to this block by a ball joint and to the clamp (22) by a shaft (31).

FIG.1

FIG.2

FIG.3

FIG.4

EP 0 471 632 B1